# EUROPÄISCHE PATENTSCHRIFT

(11) **EP 3 332 815 B1**
(45) Veröffentlichungstag und Bekanntmachung des Hinweises auf die Patenterteilung: **28.10.2020**
(21) Anmeldenummer: 17205669.9
(22) Anmeldetag: 06.12.2017
(51) Int. Cl.: B33Y 80/00, A61L 27/02, A61L 27/04, A61L 27/12, A61L 27/56, C12N 5/00, C12N 5/077

(54) **STRUKTURIERTE MINERALISCHE KNOCHENERSATZFORMKÖRPER**
STRUCTURED MINERAL BONE REPLACEMENT MOULD
ÉLÉMENT MOULÉ STRUCTURÉ DE SUBSTITUTION OSSEUSE MINÉRAL

(30) Priorität: 08.12.2016 DE 102016224453
(43) Veröffentlichungstag der Anmeldung: 13.06.2018
(73) Patentinhaber: InnoTERE GmbH, 01445 Radebeul (DE)
(72) Erfinder: NIES, Berthold, 64407 Fränkisch-Crumbach (DE)
(74) Vertreter: Kailuweit & Uhlemann Patentanwälte Partnerschaft mbB

(56) Entgegenhaltungen:
- EP-A1- 1 518 569
- WO-A2-2009/030919
- DE-B3-102013 221 575
- US-A1- 2011 151 027
- ANJA LODE ET AL: "Fabrication of porous scaffolds by three-dimensional plotting of a pasty calcium phosphate bone cement under mild conditions", JOURNAL OF TISSUE ENGINEERING AND REGENERATIVE MEDICINE, Bd. 8, Nr. 9, 30. August 2012 (2012-08-30) , Seiten 682-693, XP055169132, ISSN: 1932-6254, DOI: 10.1002/term.1563

## Beschreibung

Die Erfindung betrifft strukturierte mineralische Knochenersatzformkörper mit einem definierten interkonnektierenden Porensystem und einer vorbestimmten Struktur, ein Verfahren zur Herstellung strukturierter mineralischer Knochenersatzformkörper durch ein 3D-Druckverfahren sowie deren Verwendung zur Herstellung eines alloplastischen Implantats oder als Trägermaterial in der Zellkultur, der Gewebekultur und/oder des *Tissue Engineering ex vivo.*

In der regenerativen Medizin wird seit vielen Jahren an der Entwicklung bioresorbierbarer Implantatmaterialien geforscht, um aufwendige Operationen zur Entfernung eingesetzter Implantate zu umgehen. Bioresorbierbare Materialien besitzen die Eigenschaft, nach der Implantation im Körper allmählich durch enzymatische oder zelluläre Mechanismen im Rahmen des natürlichen Stoffwechsels abgebaut zu werden. Die dabei entstehenden Abbauprodukte werden vom Körper weitgehend resorbiert.

Herkömmliche Lösungen für die Bereitstellung von alloplastischen, d. h. künstlichen, körperfremden Implantatmaterialien basieren meist auf Calciumphosphaten, die durch Fällung oder über Hochtemperaturprozesse (Sinterung geeigneter Ausgangsmaterialien bei Temperaturen über 500°C) hergestellt werden. Eine Herstellung über Hochtemperaturprozesse liefert zwar Materialien mit stofflicher Ähnlichkeit zu Knochenmineralien, führt aber zu starken Veränderungen der Struktur, wodurch die Bioaktivität und Resorbierbarkeit eines Knochenersatzformkörpers beeinträchtigt werden.

Eine mögliche Alternative bieten mineralische Knochenzemente, die über eine hydraulische Abbindereaktion, d. h. eine Reaktion mit Wasser, *in situ* aushärten.

US 6,642,285 B1 offenbart einen hydraulischen Zement zur Herstellung von Knochenimplantaten aus drei aufeinander abgestimmten Komponenten, die nach dem Mischen möglichst direkt zu einem makroporösen Feststoff aushärten sollen. Die erste Komponente stellt eine Calciumquelle dar, die in Verbindung mit einer wässrigen Lösung und einer hydrophoben Flüssigkeit bevorzugt innerhalb von 60 Minuten vollständig aushärtet, wobei das Produkt nach der Aushärtung keine hydraulische Aktivität mehr besitzt, d. h. keine weitere Reaktion mit Wasser erfolgt.

WO 2005/084 726 A1 offenbart einen hydraulischen Knochenzement umfassend eine erste Komponente, pulverförmige Calciumphosphatpartikel, und einer zweiten Komponente, bestehend aus Wasser. Beim Mischen beider Komponenten bindet die Zusammensetzung besonders schnell innerhalb von 1 bis 20 min zu einem festen Calciumphosphatknochenzement ab.

WO 2008 148 878 A2 offenbart Pasten, Suspensionen oder Dispersionen mit einer flüssigen bis pastösen Konsistenz, bestehend aus einer resorbierbaren mineralischen Knochenzementkomponente und einer Trägerflüssigkeit, die substanziell wasserfrei sind, zur Verwendung als Knochenzemente oder Knochenersatzmaterialien, wobei die Abbindereaktion bevorzugt vollständig nach der Implantation erfolgt.

Die DE 10 2013 221 575 B3 offenbart formstabile Knochenersatzformkörper aus mineralischem Knochenzement mit verbleibender hydraulischer Aktivität, ein Verfahren zu deren Herstellung, bevorzugt durch ein (3D)-Druck-Verfahren, sowie deren Verwendung als alloplastisches Knochenimplantat. Weiterhin werden Strontiumionen als möglicher Zusatz der reaktiven mineralischen Knochenzementkomponente offenbart.

Hofmann *et al.* offenbart Calciumphosphatzemente als schnitzbare und mechanisch stabile Knochenersatzmaterialien sowie ein Verfahren zur Herstellung des Calciumphosphatknochenersatzmaterials umfassend das Anmischen eines mechanisch aktiviertem α-Tricalciumphosphat oder einer Mischung aus Tetracalcium- und Dicalciumphosphatanhydrat mit Wasser, die Formgebung in einer Gießform und das Abbinden bei 37°C für 1 bis 4,5 h (Hofmann *et al.* 2007). Der Abbruch der Abbindereaktion erfolgt durch Extraktion in Ethanol oder durch Gefriertrocknung und die vollständige Abbindung nach der Implantation durch Wasser oder Serum.

Überraschend wurde gefunden, dass so hergestellte Formkörper besonders gute mechanische Eigenschaften aufweisen, wenn die Aushärtung nicht in wässrigen Lösungen, sondern in gesättigter Wasserdampfatmosphäre bei Umgebungstemperaturen bzw. leicht erhöhten Temperaturen (25-75°C) insbesondere aber unterhalb der Sintertemperatur erfolgt.

WO 2012/101 428 A1 offenbart ein Verfahren zur Herstellung von Monetit aus einer festen Komponente, bestehend aus einem Calciumsalz, wie beispielsweise α-Tricalciumphosphat, und einer wässrigen Komponente mit einem ein- oder zweiwertigen Metallkation und einem Halogenanion bei einer Temperatur von 37°C, bevorzugt in einem Zeitraum von 36 h bis 30 min. Weiterhin offenbart WO 2012/101 428 A1 die Verwendung als Knochenersatzmaterial.

Die gängigen Verfahren zur Herstellung von porösen Knochenersatzmaterialien weisen allerdings einen geringen Einfluss auf die Anordnung der Poren und ihre Interkonnektivität auf. Beides ist von sehr großer Bedeutung für die schnelle und nachhaltige knöcherne Integration des Knochenersatzmaterials-als wichtige klinische Zielstellung.

Eine mögliche Alternative bietet das Verfahren des 3D-Drucks, welches neue technische Wege ermöglicht, definierte Porenstrukturen in porösen Knochenersatzmaterialien zu realisieren.

Lode *et al.* (Lode *et al.* 2012) offenbaren, dass wasserfreie pastöse Präparationen aus mineralischen Knochenzementen über einen Druckprozess zu porösen Formkörpern gedruckt werden können, die anschließend durch Einlegen in wässrige Lösungen zu soliden Formkörpern ausgehärtet werden können.

DE102013221575B3 offenbart formstabile Knochenersatzformkörper mit hydraulischer Aktivität durch die Verwendung schwer wasserlöslicher Trägerflüssigkeiten zum Anmischen einer formbaren Knochenzementmasse und die Formgebung bevorzugt durch ein 3D-Druckverfahren oder einen Granulationsprozess. Bei den Knochenersatzformkörpern mit verbleibender hydraulischer Aktivität erfolgt ein Teil der Abbindereaktion der zementartigen mineralischen Präparation nach der Implantation unter biologischen Bedingungen. Auf diese Weise kann eine höhere Bioaktivität durch Ausbildung einer Knochen-ähnlichen Struktur erreicht werden.

Das Verfahren des 3D-Drucks wird weiterhin bei dem sogenannten Pulverdruck angewandt. Bei diesem Verfahren werden Calciumphosphat-Pulvermischungen in dünnen Schichten ausgerakelt und an den zu verfestigenden Stellen mit reaktiven Lösungen (Abbindeflüssigkeit) kontaktiert. Dies führt lokal begrenzt zu einer Verfestigung der Pulverschicht und durch wiederholtes Ausrakeln und Kontaktieren zum Aufbau von 3D-Strukturen. Das nicht verfestigte Pulver bildet das Porensystem und wird z. B. durch Ausblasen mit Pressluft entfernt. Anschließend wird das vorverfestigte Gerüst durch einen Sinterschritt bei Temperaturen von über 500°C in einen keramischen Formkörper überführt, wodurch eine geringe Flexibilität der Zusammensetzung der zu druckenden Substanzen, aufgrund der Umwandlung oder Zerstörung im abschließenden Sinterprozess, folgt. Typische Endprodukte sind Formkörper aus Hydroxylapatit, TriCalciumphosphat oder biphasische Calciumphosphate. Nachteile dieses Verfahrens bestehen in der geringen spezifischen Oberfläche von unter 1 m²/g und der damit begrenzten Bioaktivität (auf Grund des notwendigen Sinterschritts) und den geringen Dimensionen der hergestellten Formkörper und engen Begrenzung der Porenverteilung, aufgrund mit der Größe und Komplexität zunehmender Reinigungsprobleme von nicht verfestigtem Pulver.

Daher besteht die Aufgabe der vorliegenden Erfindung darin, Knochenersatzformkörper bereitzustellen, die unter Verzicht auf einen keramischen Sinterschritt hergestellt werden und deren Formgebung unter kontrollierten Bedingungen erfolgt.
Ferner ist es Aufgabe der Erfindung, Knochenersatzformkörper bereitzustellen, welche das Knochenwachstum stimulieren sowie eine ausreichende mechanische Stabilität aufweisen.

Erfindungsgemäß wird die Aufgabe gelöst durch die strukturierten mineralischen Knochenersatzformkörper mit einem definierten interkonnektierenden Porensystem und einer vorbestimmten Struktur, hergestellt aus einem wasserfreien mineralischen Knochenzement enthaltend Calcium- und/oder Magnesiumverbindungen und 0,5 Mol-% bis 25 Mol-% Strontiumionen bezogen auf den Gesamtgehalt zweiwertiger Kationen,
wobei das definierte interkonnektierende Porensystem in mindestens einer Dimension ein interkonnektierendes Porensystem mit Poren mit einem durchschnittlichen Porenquerschnitt von mindestens 50.000 µm² und einem durchschnittlichen Porendurchmesser von mindestens 250 µm bezogen auf einen kreisrunden Querschnitt aufweist.

Erfindungsgemäß wird unter einem definierten interkonnektierenden Porensystem ein zusammenhängendes Porensystem verstanden, welches im Gegensatz zu einem statistischen Porensystem ein definiertes Muster aufweist. Unter interkonnektierend wird die Verbindung der Poren untereinander verstanden, wobei an der Verbindungsstelle zweier Poren ein Querschnitt von mindestens 50.000 µm² vorliegt.

Erfindungsgemäß wird unter einer vorbestimmten Struktur eine makroskopische Form verstanden, welche konstruierte, beliebige und komplexe Geometrien durch ein 3D-Druckverfahren annehmen kann.

In einer Ausführungsform sind die strukturierten mineralischen Knochenersatzformkörper aus mehreren aufeinander folgenden Schichten aufgebaut (gestapelt), die verschiedene geometrische Formen, wie zum Beispiel kreisförmig, oval, eckig, annehmen können.

In einer Ausführungsform sind die vorbestimmten Strukturen geometrische Formen, wie etwa Zylinder, Rechtecke, Keile, Scheiben oder an den Knochen anatomisch angepasste Konen oder Formen, bekannt von Cages für die Wirbelsäule. Unter Cages für die Wirbelsäule werden maschinell geformte Platzhalter für den Zwischenwirbelraum verstanden, welche die natürliche (physiologische) Höhe des Bandscheibensegments wieder herstellen.

Erfindungsgemäß weist das definierte interkonnektierende Porensystem mindestens in einer Dimension ein interkonnektierendes Porensystem mit Poren mit einem durchschnittlichen Porenquerschnitt von mindestens 50.000 µm² und einem durchschnittlichen Porendurchmesser von mindestens 250 µm bezogen auf einen kreisrunden Querschnitt auf.

Erfindungsgemäß wird unter einer Dimension die räumliche Abmessung eines Körpers verstanden. Die Dimension kann ausgewählt sein aus Länge, Breite oder Höhe.

Vorteilhaft können mittels 3D-Druckverfahren gezielte Porenanordnungen und gezielte Porenvolumen festgelegt werden. Vorteilhaft wird insbesondere die Gestaltung von gerichteten Poren ermöglicht, so dass der Weg für den einwachsenden neuen Knochen zur Überbrückung eines Knochendefekts auf ein Minimum verkürzt werden kann.

In einer weiteren Ausführungsform weist das definierte interkonnektierende Porensystem mindestens in zwei Dimensionen ein interkonnektierendes Porensystem mit Poren mit einem durchschnittlichen Porenquerschnitt von mindestens 50.000 µm² und einem durchschnittlichen Porendurchmesser von mindestens 250 µm bezogen auf einen kreisrunden Querschnitt auf.

In einer weiteren Ausführungsform weist das definierte interkonnektierende Porensystem in drei Dimensionen ein interkonnektierendes Porensystem mit Poren mit einem durchschnittlichen Porenquerschnitt von mindestens 50.000 µm² und einem durchschnittlichen Porendurchmesser von mindestens 250 µm bezogen auf einen kreisrunden Querschnitt auf.

In einer Ausführungsform weisen maximal 10% der Poren des definierten interkonnektierenden Porensystems einen durchschnittlichen Porenquerschnitt und einen durchschnittlichen Porendurchmesser von 50 bis 100% des durchschnittlichen Porenquerschnitts von mindestens 50.000 µm² und des durchschnittlichen Porendurchmessers von mindestens 250 µm bezogen auf einen kreisrunden Querschnitt der Poren auf.

In einer bevorzugten Ausführungsform weist das definierte interkonnektierende Porensystem in mindestens einer Dimension, bevorzugt in mindestens zwei Dimensionen, ganz besonders bevorzugt in drei Dimensionen, ein interkonnektierendes Porensystem mit Poren mit einem durchschnittlichen Porenquerschnitt von mindestens 75.000 µm² und einem durchschnittlichen Porendurchmesser von mindestens 300 µm bezogen auf einen kreisrunden Querschnitt auf.

Erfindungsgemäß weist das definierte interkonnektierende Porensystem in mindestens einer Dimension, bevorzugt in mindestens zwei Dimensionen, ganz besonders bevorzugt in drei Dimensionen, ein interkonnektierendes Porensystem mit Poren mit einem maximalen durchschnittlichen Porenquerschnitt von 785.000 µm² und einem maximalen durchschnittlichen Porendurchmesser von 1000 µm bezogen auf einen kreisrunden Querschnitt auf.

Durch die Vergrößerung des durchschnittlichen Porenquerschnitts und des durchschnittlichen Porendurchmessers erfolgt eine Verringerung der mechanischen Eigenschaften, insbesondere der Druckfestigkeit, der erfindungsgemäßen strukturierten mineralischen Knochenersatzformkörper.

In einer weiteren Ausführungsform weisen die erfindungsgemäßen strukturierten mineralischen Knochenersatzformkörper eine Gesamtporosität im Bereich von 50 bis 85% auf, bevorzugt von 60 bis 75%.

Unter Gesamtporosität wird die Summe von Makroporosität und Mikro- und Nanoporosität verstanden. Die Gesamtporosität ergibt sich aus dem Gewichtsverhältnis der ausgehärteten Formkörper zu einem Formkörper mit der theoretischen Dichte von Calciumphosphaten von 3,0 g/cm³. Unter Makroporosität wird die Summe der sichtbaren Poren mit einem Porendurchmesser von d > 100 µm verstanden und unter Mikro- und Nanoporosität wird die Summe der mikroskopisch sichtbaren Poren mit einem Porendurchmesser von d < 10µm verstanden. Die Makroporosität ergibt sich durch die Formgebung der Knochenzementmasse und beeinflusst das Einwachsen von Zellen und Gewebestrukturen in die Knochenersatzformkörper. Die Mikro- und Nanoporosität ergibt sich aus der Dichte der Knochenzementmasse und beeinflusst die spezifische Oberfläche, welche im Zusammenhang mit der Adsorption von Biomolekülen und der Interaktion mit Knochenzellen steht.

In einer weiteren Ausführungsform weisen die erfindungsgemäßen strukturierten mineralischen Knochenersatzformkörper eine Makroporosität im Bereich zwischen 30 und 65% und eine Mikroporosität im Bereich zwischen 20 und 35% auf.

In einer weiteren Ausführungsform weisen die erfindungsgemäßen strukturierten mineralischen Knochenersatzformkörper eine spezifische Oberfläche von mindestens 5 m²/g auf, bevorzugt zwischen 5 und 50 m²/g auf.

Die spezifische Oberfläche der erfindungsgemäßen strukturierten mineralischen Knochenersatzformkörper kann über BET-Messung und Quecksilberporosimetrie bestimmt werden.

Unter BET-Messung (Brunauer-Emmett-Teller) wird ein Analyseverfahren zur Größenbestimmung von Oberflächen mittels Gasadsorption verstanden, bei dem die massenbezogene spezifische Oberfläche aus experimentellen Daten berechnet wird.

Unter Quecksilberporosimetrie wird ein Analyseverfahren zur Größenbestimmung von Oberflächen mittels einer nichtbenetzenden Flüssigkeit, wie Quecksilber, verstanden. Die Porengröße wird als Funktion des äußeren Drucks gemessen, der notwendig ist, um die Flüssigkeit in eine Pore gegen die Oberflächenspannung der Flüssigkeit zu drücken und kann durch die Washburn-Gleichung beschrieben werden (Washburn, 1921). Aus den so genannten Intrusions- und Extrusionskurven erfolgt die Berechnung der Porengrößenverteilung.

Erfindungsgemäß wird unter einem wasserfreien mineralischen Knochenzement ein mineralischer Knochenzement verstanden, welcher ohne Zusatz von Wasser angemischt wird. Unter einem reaktiven mineralischen Knochenzement wird die mindestens eine reaktive mineralische oder organomineralische Feststoffkomponente (Knochenzementkomponente) verstanden, die bei dem Kontaktieren mit einer wässrigen Lösung oder nach Einbringung in eine wässrige Lösung in einem Abbindeprozess zu einem schwerlöslichen Festkörper abbinden kann.

Mineralische Knochenzemente bestehen aus mindestens einer reaktiven mineralischen oder organomineralischen Feststoffkomponente (Knochenzementkomponente), die bei dem Kontaktieren mit einer wässrigen Lösung oder nach Einbringung in eine wässrige Lösung in einem Abbindeprozess zu einem schwerlöslichen Festkörper abbinden kann.

Erfindungsgemäß werden die strukturierten mineralischen Knochenersatzformkörper aus einem wasserfreien mineralischen Knochenzement hergestellt, enthaltend Calciumverbindungen und 0,5 Mol-% bis 25 Mol-% Strontiumverbindungen, bezogen auf den Gesamtgehalt zweiwertiger Kationen, oder Magnesiumverbindungen und 0,5 Mol-% bis 25 Mol-% Strontiumverbindungen, bezogen auf den Gesamtgehalt zweiwertiger Kationen, oder Calciumverbindungen und Magnesiumverbindungen und 0,5 Mol-% bis 25 Mol-% Strontiumverbindungen, bezogen auf den Gesamtgehalt zweiwertiger Kationen.

Bevorzugt werden die strukturierten mineralischen Knochenersatzformkörper aus einem wasserfreien mineralischen Knochenzement hergestellt, enthaltend 2 Mol-% bis 20 Mol-% Strontiumionen, bezogen auf den Gesamtgehalt zweiwertiger Kationen, besonders bevorzugt 5 Mol-% bis 15 Mol-% Strontiumionen.

Vorteilhaft werden das Knochenwachstum auf den strukturierten mineralischen Knochenersatzformkörper durch die Strontiumionen sowie die mechanischen Eigenschaften, insbesondere die Druckfestigkeit, der strukturierten mineralischen Knochenersatzformkörper durch die Strontiumionen verbessert.

In einer Ausführungsform weist der strukturierte mineralische Knochenersatzformkörper eine verbleibende hydraulische Aktivität auf.

Hydraulische Aktivität bezeichnet im Sinne der Erfindung die qualitative Fähigkeit eines erfindungsgemäßen strukturierten mineralischen Knochenersatzformkörpers, durch die Zugabe von Wasser und/oder einer wasserhaltigen Komponente eine chemische Reaktion, d. h. einen Abbindeprozess, einzugehen.

In einer weiteren Ausführungsform enthalten die strukturierten mineralischen Knochenersatzformkörper mit verbleibender hydraulischer Aktivität einen Anteil an abgebundenem mineralischem Knochenzement von 5 Gew.-% bis 90 Gew.-%, besonders bevorzugt von 10 Gew.-% bis 80 Gew.-%, ganz besonders bevorzugt von 30 Gew.-% bis 70 Gew.-%.

Der Anteil an abgebundenem mineralischem Knochenzement kann mittels Röntgendiffraktometrie (XRD) bestimmt werden. Zur Quantifizierung kann die Rietveld-Methode verwendet werden. Die Rietveld-Methode dient der quantitativen Phasenanalyse, also der quantitativen Bestimmung der kristallinen Komponenten einer pulverförmigen Probe.

In einer Ausführungsform wird der Abbindeprozess bzw. die chemische Reaktion mit Wasser und/oder einer wasserhaltigen Komponente durch Wasserentzug vor Erreichen der vollständigen Umsetzung der reaktiven mineralischen Knochenzementkomponenten unterbrochen.

In einer weiteren Ausführungsform erfolgt durch den Kontakt eines erfindungsgemäßen strukturierten mineralischen Knochenersatzformkörpers mit verbleibender hydraulischer Aktivität mit Wasser und/oder einer wasserhaltigen Komponente eine Hydratation der verbleibenden reaktiven mineralischen Knochenzementkomponente, wodurch der unterbrochene Abbindeprozess fortgeführt wird.

In einer weiteren Ausführungsform erfolgt der erneute Kontakt eines erfindungsgemäßen strukturierten mineralischen Knochenersatzformkörpers mit verbleibender hydraulischer Aktivität mit Wasser und/oder einer wasserhaltigen Komponente erst unmittelbar vor dessen Verwendung oder während oder nach seiner Verwendung, also während oder nach der Implantation in den Körper.

Vorteilhaft weisen die strukturierten mineralischen Knochenersatzformkörper mit verbleibender hydraulischer Aktivität eine hohe biologische Aktivität auf. Erfindungsgemäß bezeichnet biologische Aktivität die Eigenschaft bei der Aushärtung unter biologischen Bedingungen eine dem Knochen ähnliche Kristallstruktur zu bilden, wodurch eine Interaktion mit den organischen und zellulären Elementen des sich bildenden neuen Knochens ermöglicht wird. Je höher die zum Anwendungszeitpunkt verbleibende hydraulische Aktivität ist, desto höher ist die biologische Aktivität. Abgestimmt auf die Anforderungen des jeweiligen klinischen Einsatzgebiets kann so ein strukturierter mineralischer Knochenersatzformkörper mit vorbestimmter Druckfestigkeit und biologischer Aktivität bereitgestellt werden.

In einer Ausführungsform ist der strukturierte mineralische Knochenersatzformkörper vollständig ausgehärtet bzw. vollständig abgebunden, d. h. weist keine hydraulische Aktivität auf. Dies ist dann der Fall, wenn eine vergleichsweise hohe mechanische Stabilität angestrebt wird.

In einer Ausführungsform weisen die vollständig abgebundenen, strukturierten mineralischen Knochenersatzformkörper Druckfestigkeiten von mindestens 10 MPa auf.

Erfindungsgemäß wird unter Druckfestigkeit die Widerstandsfähigkeit eines Werkstoffs bei der Einwirkung von Druckkräften bezeichnet. Die Druckfestigkeit ist der Quotient aus Bruchlast und Querschnittsfläche A eines Prüfkörpers. Sie wird ausgedrückt als Kraft pro Fläche (in Pa = N/m²). Ist die anliegende Druckspannung größer als die Druckfestigkeit eines Körpers, so wird er zerstört. Weiterhin wird unter Druckfestigkeit die uniaxiale Druckfestigkeit verstanden. Die Druckfestigkeit kann an definierten Formkörpern mit planparallelen Ober- und Unterseiten auf einer Universaltestmaschine (Fa. Hegewald und Peschke) mittels einer Kraftmessdose mit 20 kN Maximalbelastung und einem Vorschub von 1 mm/min bestimmt werden.

In einer Ausführungsform weisen die vollständig abgebundenen, strukturierten mineralischen Knochenersatzformkörper Druckfestigkeiten im Bereich von 10 bis 100 MPa, bevorzugt im Bereich von 10 bis 50 MPa, besonders bevorzugt im Bereich von 10 bis 25 MPa, auf.

In einer weiteren Ausführungsform weist der strukturierte mineralische Knochenersatzformkörper mit verbleibender hydraulischer Aktivität mindestens 5% der Druckfestigkeit eines vollständig abgebundenen Knochenersatzformkörpers aus den gleichen mineralischen Knochenzementkomponenten und mit dem gleichen definierten interkonnektierenden Porensystem und der gleichen vorbestimmten Struktur auf. Vorteilhaft weist ein strukturierter mineralischer Knochenersatzformkörper mit verbleibender hydraulischer Aktivität und mindestens 5% der Druckfestigkeit eines vollständig abgebundenen Knochenersatzformkörpers eine ausreichende Formstabilität auf, um den mechanischen Belastungen, die z. B. mit Lagerung und Transport einhergehen, standhalten zu können.

Weiterhin wird die Aufgabe gelöst durch ein Verfahren zur Herstellung strukturierter mineralischer Knochenersatzformkörper, umfassend die folgenden Schritte:
a) Anmischen eines reaktiven mineralischen Knochenzementes umfassend Calcium- und/oder Magnesiumverbindungen und 0,5 Mol-% bis 25 Mol-% Strontiumverbindungen bezogen auf den Gesamtgehalt zweiwertiger Kationen mit einer wasserfreien Trägerflüssigkeit zu einer formbaren Knochenzementmasse,
b) Formgebung der Knochenzementmasse durch ein 3D-Druckverfahren zu einem strukturierten mineralischen Knochenersatzformkörper mit einer vorbestimmten Struktur und einem definierten interkonnektierenden Porensystem,
   wobei der Durchmesser der Stränge der Knochenzementmasse zwischen 200 µm und 1200 µm, bevorzugt zwischen 250 µm und 1000 µm und besonders bevorzugt zwischen 300 µm und 800 µm, beträgt und
   wobei der Abstand der Stränge der Knochenzementmasse zwischen 200 µm und 1500 µm, bevorzugt zwischen 250 µm und 1000 µm und besonders bevorzugt zwischen 300 µm und 800 µm, beträgt,
c) Initiation des Abbindeprozesses durch Kontaktieren des Knochenersatzformkörpers mit einer wässrigen Lösung oder einer Wasserdampf-gesättigten Umgebung,
d) Abbruch des Abbindeprozesses durch den substantiellen Entzug von Wasser.

Vorteilhaft werden durch das erfindungsgemäße Verfahren unter Verzicht auf einen keramischen Sinterschritt strukturierte mineralische Knochenersatzformkörper hergestellt, deren Formgebung unter kontrollierten Bedingungen erfolgt, die über einen hydraulischen Abbindeprozess soweit verfestigt werden, dass eine ausreichende Formstabilität für Lagerung, Transport und die jeweiligen Implantationsbedingungen gewährleistet ist, wobei die Knochenersatzformkörper vor, während und/oder nach der Implantation vollständig aushärten bzw. ausgehärtet werden.

Erfindungsgemäß umfasst der reaktive mineralische Knochenzement Calciumverbindungen und 0,5 Mol-% bis 25 Mol-% Strontiumverbindungen, bezogen auf den Gesamtgehalt zweiwertiger Kationen, oder Magnesiumverbindungen und 0,5 Mol-% bis 25 Mol-% Strontiumverbindungen, bezogen auf den Gesamtgehalt zweiwertiger Kationen, oder Calciumverbindungen und Magnesiumverbindungen und 0,5 Mol-% bis 25 Mol-% Strontiumverbindungen, bezogen auf den Gesamtgehalt zweiwertiger Kationen.

In einer Ausführungsform des erfindungsgemäßen Verfahrens wird eine homogene formbare Knochenzementmasse in Form einer pastösen Dispersion durch intensives Vermischen mindestens einer pulverförmigen reaktiven mineralischen Knochenzementkomponente mit einer wasserfreien Trägerflüssigkeit erhalten.

In einer Ausführungsform des erfindungsgemäßen Verfahrens wird als reaktiver mineralischer Knochenzement mindestens eine Substanz aus der Gruppe der Silikate, Phosphate, Sulfate, Carbonate, Oxide und/oder Hydroxide in Verbindung mit Calciumionen und Strontiumionen oder Magnesiumionen und Strontiumionen oder Calciumionen und Magnesiumionen und Strontiumionen verwendet.

In einer weiteren Ausführungsform des erfindungsgemäßen Verfahrens sind die Strontiumverbindungen ausgewählt aus Strontiumcarbonaten, Strontiumoxiden, Strontiumhydroxiden und/oder Strontiumphosphaten, bevorzugt sind die Strontiumverbindungen ausgewählt aus SrCO₃, SrHPO₄, Sr₂P₂O₇, Sr₂(PO₄)₂ und Sr₅(PO₄)₃OH.

In einer weiteren Ausführungsform des erfindungsgemäßen Verfahrens wird als reaktiver mineralischer Knochenzement mindestens eine Substanz, ausgewählt aus Calciumphosphaten, Calciumcarbonaten, Calciumoxiden, Calciumhydroxiden, Calciumsulfaten, Calciumsilikaten, Magnesiumphosphaten, Magnesiumcarbonaten, Magnesiumhydroxidcarbonaten, Magnesiumoxiden und analoge Verbindungen, die Calcium und Magnesium in unterschiedlichen Mol-Verhältnissen (insbesondere Calcium-Magnesium-Phosphate und Calcium-Magnesiumcarbonate) verwendet. Zusätzlich können wasserlösliche Alkali- oder Ammoniumphosphate sowie Alkali- oder Ammoniumcitrate, Alkali- oder Ammoniumtartrate oder Alkalisilikate als Reaktionspartner oder zur Beeinflussung der Abbindereaktion enthalten sein.

In einer bevorzugten Ausführungsform des erfindungsgemäßen Verfahrens wird als reaktiver mineralischer Knochenzement mindestens eine Substanz aus der Gruppe der Phosphate in Verbindung mit Calcium-, Magnesium- oder Strontiumionen, einschließlich Hydrogen- und Dihydrogenphosphate, verwendet.

In einer weiteren Ausführungsform des erfindungsgemäßen Verfahrens wird als reaktiver mineralischer Knochenzement mindestens ein Phosphat in Verbindung mit Calciumionen verwendet, wobei das molare Verhältnis Calcium zu Phosphat mindestens 1 und das molare Verhältnis zweiwertiger Kationen zu Phosphationen mindestens 1,35 ist.

In einer weiteren Ausführungsform des erfindungsgemäßen Verfahrens werden als reaktiver mineralischer Knochenzement Calcium- und/oder Magnesiumsalze der Orthophosphorsäure verwendet.

In einer weiteren Ausführungsform des erfindungsgemäßen Verfahrens werden als reaktiver mineralischer Knochenzement Mischungen aus Calciumphosphaten und/oder Magnesiumphosphaten mit Carbonaten, Oxiden oder Hydroxiden des Calciums, Magnesiums oder Strontiums, oder Mischungen aus Carbonaten, Oxiden oder Hydroxiden des Calciums, Magnesiums oder Strontiums mit Alkaliphosphaten (mono-, di- und tri-Alkaliphosphaten), mono- und di-Ammoniumphosphaten oder Alkalisilikaten verwendet.

Erfindungsgemäß umfasst der reaktive mineralische Knochenzement weiterhin eine wasserfreie Trägerflüssigkeit.

Unter einer Trägerflüssigkeit wird eine organische Trägerflüssigkeit verstanden, in der der reaktive mineralische Knochenzement dispergiert ist, wobei keine chemische Reaktion stattfindet Unter einer wasserfreien Trägerflüssigkeit wird eine organische Trägerflüssigkeit verstanden, welche einen Wassergehalt von maximal 0,5% (v/v) hat.

Erfindungsgemäß ist die wasserfreie Trägerflüssigkeit bioverträglich, bevorzugt sind Trägerflüssigkeiten, die in Medizinprodukten und pharmazeutischen Produkten bereitseingesetzt werden (insbesondere als Hilfsstoffe) oder Substanzen, die im Körper unter natürlichen Bedingungen vorkommen, besonders bevorzugt sind zugelassene pharmazeutische Hilfsstoffe, insbesondere solche, die für parenterale Anwendung zugelassen sind, d. h. sterile Zubereitungen, die zur Injektion, Infusion oder Implantation in den menschlichen oder tierischen Körper bestimmt sind.

Die wasserfreie Trägerflüssigkeit ist ausgewählt aus Isopropylmyristat, kurz- und mittelkettigen Triglyceriden und kurz- und mittelkettigen Fettsäureestern des Glykols.

Unter kurzkettigen Triglyceriden oder Fettsäureestern werden Verbindungen von Fettsäuren einer Länge von jeweils 2 bis 5 Kohlenstoffatomen verstanden. Unter mittelkettigen Triglyceriden oder Fettsäureestern werden Verbindungen von Fettsäuren einer Länge von jeweils 6 bis 14 Kohlenstoffatomen verstanden.

Bevorzugt ist die wasserfreie Trägerflüssigkeit ausgewählt aus kurz- oder mittelkettigen Triglyceriden, mittelkettigen Fettsäureestern des Ethylenglykols und des Propylenglykols, Polymeren des Ethylenglykols, kurzkettigen Oligomeren des Propylenglykols, Co-Polymeren mit Ethylenglykol und Propylenglykoleinheiten, Mono- und Di-Methylethern des Polyethylenglykols, Glycerin und dessen wasserlöslichen Ethern und Estern und Di- und Polyglycerin.

Besonders bevorzugt ist die wasserfreie Trägerflüssigkeit ausgewählt aus Ester aus Triglyceriden mit Fettsäuren, die durchschnittlich weniger als 14 C-Atome aufweisen, Polypropylenglykole und Ester von Polypropylenglykolen sowie Mono- und Diether von Polypropylenglykolen mit Monoalkoholen und Polyethylenglykole (PEGs) mit einem Molekulargewicht von < 1000 sowie deren Mono- und Diether.

In einer weiteren Ausführungsform des erfindungsgemäßen Verfahrens beträgt der Gewichtsanteil an wasserfreier Trägerflüssigkeit bezogen auf die Gesamtmasse des reaktiven mineralischen Knochenzementes 5 Gew.-% bis 25 Gew.-%, bevorzugt 5 Gew.-% bis 20 Gew.-%, besonders bevorzugt 5 Gew.-% bis 17,5 Gew.-%.

In einer weiteren Ausführungsform des erfindungsgemäßen Verfahrens enthält die formbare Knochenzementmasse weitere Füllstoffe und/oder Wirkstoffe, und pharmakologische Wirkstoffe, die an der Abbindereaktion nicht teilnehmen.

In einer weiteren Ausführungsform des erfindungsgemäßen Verfahrens enthält die formbare Knochenzementmasse oberflächenaktive Substanzen, welche aus der Gruppe der Tenside ausgewählt sind.

In einer weiteren Ausführungsform des erfindungsgemäßen Verfahrens enthält die formbare Knochenzementmasse mindestens zwei Tenside, ausgewählt aus mindestens zwei der Gruppen der anionischen, kationischen, amphoteren und nichtionischen Tenside.

In einer bevorzugten Ausführungsform des erfindungsgemäßen Verfahrens enthält die formbare Knochenzementmasse mindestens ein anionisches und mindestens ein nichtionisches Tensid.

In einer weiteren Ausführungsform des erfindungsgemäßen Verfahrens ist das anionische Tensid ausgewählt aus Fettsäuren und deren Salzen, Estern von Fettsäuren und deren Salzen, Carbonsäureethern, Phosphorsäureestern und Salzen davon, Acylaminosäuren und Salzen davon, bevorzugt aus einem mit Zitronensäure, Schwefelsäure oder Phosphorsäure veresterten Fettalkohol, einem mit Zitronensäure, Schwefelsäure oder Phosphorsäure veresterten Mono- oder Diglycerid oder Salzen davon oder mindestens einer mit Zitronensäure, Schwefelsäure oder Phosphorsäure veresterten Fettsäure oder Salze davon.

In einer weiteren Ausführungsform des erfindungsgemäßen Verfahrens ist das nichtionische Tensid ausgewählt aus Fettalkoholen, ethoxylierten Fettalkoholen, Ethylenoxid/Propylenoxid-Blockcopolymeren, ethoxylierten Fetten und Ölen, Polyethylenglykolfettsäureestern, Sorbitanestern, ethoxylierten Sorbitanestern, Polyglycerinmonoestern und Aminoxiden, bevorzugt aus einem ethoxylierten Fettalkohol, einer ethoxylierten Fettsäure, einem ethoxylierten Sorbitanfettsäureester, besonders bevorzugt Polysorbat 80; einem ethoxylierten Fett oder einem ethoxylierten Öl, besonders bevorzugt polyethoxyliertes Castoröl.

In einer weiteren Ausführungsform des erfindungsgemäßen Verfahrens beträgt die Gesamtmasse der nichtionischen Tenside mindestens das Doppelte der Gesamtmasse der anionischen Tenside.

Vorteilhaft unterstützen oder ermöglichen oberflächenaktive Substanzen (Tenside) das Eindringen von Wasser oder Wasserdampf oder Luftfeuchtigkeit in die geformte Knochenzementmasse bzw. den strukturierten mineralischen Knochenersatzformkörper mit hydraulischer Aktivität zur Auslösung und zum Fortschreiten der Abbindereaktion.

In einer weiteren Ausführungsform des erfindungsgemäßen Verfahrens ist ein weiterer Bestandteil des reaktiven mineralischen Knochenzementes mindestens ein Abbindebeschleuniger.

Vorteilhaft werden durch Abbindebeschleuniger die Abbindezeit und der pH-Wert-Verlauf bei der Abbindereaktion des reaktiven mineralischen Knochenzementes eingestellt.

In einer weiteren Ausführungsform des erfindungsgemäßen Verfahrens ist der mindestens eine Abbindebeschleuniger ausgewählt aus Phosphatsalzen, organischen Säuren oder Salzen organischer Säuren, bevorzugt aus Natrium- und/oder Kaliumionen- oder Ammoniumionen-haltigen Phosphaten oder Natrium- und/oder Kaliumionen- oder Ammoniumionen-haltigen Salzen organischer Säuren und deren Mischungen untereinander, besonders bevorzugt Hydrogenphosphate, ganz besonders bevorzugt Ammonium-, Natrium- oder Kaliumhydrogenphosphat.

In einer weiteren Ausführungsform des erfindungsgemäßen Verfahrens ist der mindestens eine Abbindebeschleuniger mit einem Massenanteil (in Bezug auf die Masse des mineralischen Zementpulvers) von 0,1% bis 5%, besonders bevorzugt 0,2% bis 4%, ganz besonders bevorzugt 0,5% bis 3,5% in dem erfindungsgemäßen reaktiven mineralischen Knochenzement enthalten.

In einer weiteren Ausführungsform des erfindungsgemäßen Verfahrens ist ein weiterer Bestandteil des reaktiven mineralischen Knochenzementes mindestens ein pharmakologischer Wirkstoff.

In einer weiteren Ausführungsform des erfindungsgemäßen Verfahrens sind die pharmakologischen Wirkstoffe ausgewählt aus Antibiotika, Antiseptika, antimikrobiellen Peptiden oder antimikrobiellen Proteinen, Nukleinsäuren, bevorzugt siRNA; antiresorptiven Wirkstoffen, bevorzugt Bisphosphonaten, Kortikoiden, Fluoriden, Protonenpumpen-Inhibitoren; Parathormon und dessen Derivaten, knochenwachstums-stimulierenden Wirkstoffen, bevorzugt Wachstumsfaktoren, Vitaminen, Hormonen, Morphogenen, besonders bevorzugt knochenmorphogenetische Proteine und Peptide; sowie angiogene Wirkstoffe, bevorzugt Fibroblasten-Wachstumsfaktoren wie aFGF, bFGF oder FGF18; Entzündungshemmer und Antitumorsubstanzen.

Vorteilhaft eignen sich die strukturierten mineralischen Knochenersatzformkörper besonders als Träger für pharmakologische Wirkstoffe, da durch die Resorption des mineralischen Knochenzements eine sukzessive Wirkstofffreisetzung erreicht wird.

In einer weiteren Ausführungsform des erfindungsgemäßen Verfahrens ist ein weiterer Bestandteil des reaktiven mineralischen Knochenzementes mindestens ein Füllstoff ausgewählt aus mineralischen und organischen Substanzen.

In einer weiteren Ausführungsform des erfindungsgemäßen Verfahrens ist der mindestens eine Füllstoff wasserlöslich. Vorteilhaft wird durch die Verwendung wasserlöslicher Füllstoffe die Porosität des bei dem Abbindeprozess mit Wasser gebildeten Feststoffs eingestellt.

In einer weiteren Ausführungsform des erfindungsgemäßen Verfahrens sind wasserlösliche Füllstoffe ausgewählt aus Zuckern, bevorzugt Saccharose; Zuckeralkoholen, bevorzugt Sorbit, Xylit, Mannit; wasserlöslichen Salzen, bevorzugt Natriumchlorid, Natriumcarbonat, Ammoniumcarbonat oder Calciumchlorid.

In einer weiteren Ausführungsform des erfindungsgemäßen Verfahrens weist der wasserlösliche Füllstoff eine Partikelgröße von 10 µm bis 2000 µm, bevorzugt von 100 µm bis 1000 µm, auf.

In einer weiteren Ausführungsform des erfindungsgemäßen Verfahrens enthält der reaktive mineralische Knochenzement 5 Vol.-% bis 90 Vol.-% wasserlösliche Füllstoffe, bevorzugt 10 Vol.-% bis 80 Vol.-%, bezogen auf das Gesamtvolumen des reaktiven mineralischen Knochenzements.

In einer weiteren Ausführungsform des erfindungsgemäßen Verfahrens ist ein weiterer Bestandteil des reaktiven mineralischen Knochenzementes mindestens ein polymerer Zusatz ausgewählt aus Chitosan, Hyaluronsäure, Gelatine, Kollagen, Chondroitinsulfat, Cellulosederivaten, Stärkederivaten, Alginat, wasserlöslichen Acrylaten, Polyethylenglycol, Polyethylenoxid, PEG-PPG-Copolymeren, Polyvinylpyrrolidon und Copolymeren aus wasserlöslichen Acrylaten mit Polyethylenglycol und/oder Polyethylenoxid.
In einer weiteren Ausführungsform des erfindungsgemäßen Verfahrens sind sämtliche Bestandteile des reaktiven mineralischen Knochenzementes resorbierbar.

Erfindungsgemäß erfolgt die Formgebung der Knochenzementmasse durch ein 3D-Druckverfahren zu einem strukturierten mineralischen Knochenersatzformkörper. Unter 3D-Druckverfahren versteht man ein Extrusions-basiertes Druckverfahren, bei dem pastöse oder hochviskose Materialien durch Dosiernadeln ausgegeben und als Stränge in aufeinander folgenden Schichten (gestapelt) zu dreidimensionalen Objekten abgelagert werden.

Die Formgebung der Knochenzementmasse erfolgt durch ein 3D-Druckverfahren zu einem strukturierten mineralischen Knochenersatzformkörper mittels eines *computer-aided design (CAD)-*/*computer-aided manufacturing* (CAM)-Prozess.

Unter CAD-/CAM-Prozess wird die Kombination von Rechner-unterstütztem Konstruieren, d. h. der Entwurf und die Konstruktion eines virtuellen Modells dreidimensionaler Objekte mit Hilfe eines Computers, mit einer Rechner-unterstützten Fertigung verstanden.

Vorteilhaft wird mittels des 3D-Drucks hoch flexibel ein strukturierter mineralischer Knochenersatzformkörper mit einer vorbestimmten Struktur und einem definierten interkonnektierenden Porensystem hergestellt.

In einer Ausführungsform erfolgt die Formgebung der Knochenzementmasse durch den 3D-Druck zu definierten Mustern bzw. einem definierten interkonnektierenden Porensystem durch die Variation der Lage der Stränge, der Abstände der Stränge, der Orientierung der Schichten zueinander (Winkel) sowie die mehrlagige Ablage gleicher Strangebenen.

In einer weiteren Ausführungsform erfolgt die Formgebung der Knochenzementmasse durch die Ablagerung von 4 bis 1.000 Schichten, bevorzugt 10 bis 500 Schichten, ganz besonders bevorzugt 10 bis 100 Schichten.

In einer weiteren Ausführungsform erfolgt die Formgebung der Knochenzementmasse durch die Ablagerung von Schichten in einer Orientierung in einem Winkel zwischen 0°und 90° im Vergleich zu der unterliegenden Schicht.

In einer weiteren Ausführungsform erfolgt ein Wechsel der Orientierung der Schichten nach jeder Schicht oder jeder zweiten Schicht.
In einer weiteren Ausführungsform erfolgt ein Wechsel der Orientierung der Schichten zueinander in einem Winkel zwischen 0° und 90°, bevorzugt 15°, 45°, 60° oder 90°.

In einer weiteren Ausführungsform erfolgt eine alternierende Ablagerung von Strängen in einer Orientierung in einem Winkel von 90° im Vergleich zu der unterliegenden Schicht.

In einer weiteren Ausführungsform erfolgt bei mindestens 50 Vol-% des strukturierten mineralischen Knochenersatzformkörpers eine alternierende Ablagerung von Strängen in einer Orientierung in einem Winkel von 90° im Vergleich zu der unterliegenden Schicht.

In einer weiteren Ausführungsform erfolgt das Anbringen von Löchern für Befestigungselemente, indem an diesen Stellen keine Stränge abgelegt werden oder die Strangablage um das Loch herum erfolgt und gegebenenfalls weitere Strangablage zur Materialverstärkung erfolgt.

Vorteilhaft wird durch das interkonnektierende Porensystem eine möglichst schnelle und vollständige knöcherne Integration erreicht, da die Strecke für den Knocheneinwuchs aufgrund des geradlinigen Porensystems minimal ist.

Erfindungsgemäß erfolgt die Initiation des Abbindeprozesses durch Kontaktieren des Knochenersatzformkörpers mit einer wässrigen Lösung oder einer gesättigten Wasserdampfatmosphäre.

In einer weiteren Ausführungsform erfolgt die Initiation des Abbindeprozesses in einer gesättigten Wasserdampfatmosphäre bei mindestens 90% relativer Luftfeuchtigkeit.

In einer weiteren Ausführungsform erfolgt die Initiation des Abbindeprozesses bei einer Temperatur zwischen 0°C und 150°C, bevorzugt zwischen 25°C und 75°C.

In einer weiteren Ausführungsform erfolgt der Abbindeprozess in mehreren Stufen, durch Initiation des Abbindeprozesses in gesättigter Wasserdampfatmosphäre und Fortsetzung des Abbindeprozesses in mindestens einem weiteren Schritt in mindestens einer wässrigen Lösung. Die Schritte können unmittelbar aufeinander folgen oder in zeitlichem Abstand.
In einer weiteren Ausführungsform enthält die wässrige Lösung zur Initiation oder Fortsetzung des Abbindeprozesses mindestens einen Zusatzstoff, ausgewählt aus einer Pufferlösung, einem organischen und/oder anorganischen Salz, einem Protein, einer Zellpräparation, einem biologischen, rekombinanten oder pharmakologischen Wirkstoff, einer Nukleinsäure, einer Mischung von Nukleinsäuren, einer Aminosäure, einer modifizierten Aminosäure, einem Vitamin und einer Mischung aus diesen.

In einer weiteren Ausführungsform erfolgt der Abbindeprozess in einer gesättigten Wasserdampfatmosphäre bei mindestens 90% relativer Luftfeuchtigkeit für 12 h bis 7 d, bevorzugt 18 h bis 4 d. In einer weiteren Ausführungsform erfolgt der Abbindeprozess bei einer Temperatur zwischen 20°C und 80°C, bevorzugt 37°C bis 50°C.

In einer weiteren Ausführungsform erfolgt mindestens ein Teil des Abbindeprozesses unter erhöhter Temperatur (> 100°C) und unter erhöhtem Druck (> 1 bar) im Autoklaven und dient gleichzeitig der Sterilisation der strukturierten mineralischen Knochenersatzformkörper.

In einer Ausführungsform erfolgt der Abbruch des Abbindeprozesses durch den substantiellen Entzug von Wasser bei einer Temperatur zwischen 0°C und 150°C durch ein Trocknungsverfahren oder durch einen Austausch des Wassers gegen mindestens ein leicht flüchtiges, toxikologisch unbedenkliches Lösungsmittel, bevorzugt ausgewählt aus Aceton und niederen Alkoholen, ganz besonders bevorzugt Aceton.

Unter Wasser, das substanziell entzogen werden kann, wird erfindungsgemäß physikalisch gebundenes oder kondensiertes Wasser verstanden.

Unter niederen Alkoholen werden gesättigte Kohlenwasserstoffe mit einer Hydroxylgruppe (OH-Gruppe) verstanden, welche maximal 10 Kohlenstoffatome (C-Atome) aufweisen, wie Methanol, Ethanol, Isopropanol oder Propanol. Bevorzugt ist der niedere Alkohol Ethanol.

Vorteilhaft ist das leicht flüchtige, toxikologisch unbedenkliche Lösungsmittel mit Wasser mischbar und kann die wasserfreie Trägerflüssigkeit lösen. Vorteilhaft erfolgen durch einen Austausch des Wassers gegen mindestens ein leicht flüchtiges, toxikologisch unbedenkliches Lösungsmittel der substantielle Entzug von Wasser sowie die vollständige Entfernung der wasserfreien Trägerflüssigkeit und anderer Hilfsstoffe, wobei der Fachmann das leicht flüchtige, toxikologisch unbedenkliche Lösungsmittel entsprechend der Löslichkeitseigenschaften der eingesetzten wasserfreien Trägerflüssigkeit und der Hilfsstoffe auswählen wird. Ein partieller Verbleib der Trägerflüssigkeit und/oder der Hilfsstoffe im erfindungsgemäßen strukturierten mineralischen Knochenersatzformkörper wird dadurch nicht ausgeschlossen. Vorteilhaft ermöglicht der Einsatz biokompatibler Stoffe als Trägerflüssigkeiten und Hilfsstoffe den Einsatz derartiger strukturierter mineralischer Knochenersatzformkörper im menschlichen Körper.

In einer weiteren Ausführungsform folgt dem substantiellen Entzug von Wasser durch den Austausch des Wassers gegen mindestens ein leicht flüchtiges, toxikologisch unbedenkliches Lösungsmittel ein Trocknungsverfahren, um enthaltene Hilfsstoffe, Restwasser und das Lösungsmittel zu entfernen.

In einer Ausführungsform erfolgt der Abbruch des Abbindeprozesses durch ein Trocknungsverfahren, wobei eine Variation physikalischer Umgebungsparameter erfolgt, bevorzugt durch eine Verringerung des Drucks und/oder eine Erhöhung der Temperatur oder eine Gefriertrocknung.

In einer weiteren Ausführungsform erfolgt der substantielle Entzug von Wasser durch dessen Verbrauch in der chemischen Abbindereaktion, wobei der vorhandene Anteil an physikalisch gebundenem Wasser vollständig in chemisch gebundenes Wasser umgewandelt wird. In dieser Ausführungsform wird bei der Initiation des Abbindeprozesses weniger Wasser zur Verfügung gestellt, als für die vollständige Abbindereaktion erforderlich wäre.

In einer weiteren Ausführungsform erfolgt eine Unterbrechung des substantiellen Entzugs von Wasser, bevor die Abbindereaktion vollständig abgeschlossen ist, wobei ein strukturierter mineralischer Knochenersatzformkörper mit einer verbleibenden hydraulischen Aktivität entsteht.

Vorteilhaft sind die erfindungsgemäßen strukturierten mineralischen Knochenersatzformkörper vor dem ersten Kontaktieren mit einer wässrigen Lösung oder einer gesättigten Wasserdampfatmosphäre plastisch verformbar, aber die strukturierten mineralischen Knochenersatzformkörper mit einer verbleibenden hydraulischen Aktivität formstabil, wodurch der Knochenersatzformkörper mit verbleibender hydraulischer Aktivität eine ausreichende mechanische Festigkeit für den Transport, die Lagerung und die Implantation aufweist. Die Härte und Druckfestigkeit der Knochenersatzformkörper mit verbleibender hydraulischer Aktivität kann noch signifikant unter der eines vollständig abgebundenen Knochenersatzformkörpers aus den gleichen mineralischen Knochenzementkomponenten und mit dem gleichen definierten interkonnektierenden Porensystem und der gleichen vorbestimmten Struktur liegen, wodurch vorteilhaft die Formgebung und/oder Strukturierung eines Implantats auf der Basis eines erfindungsgemäßen Knochenersatzformkörpers zur formschlüssigen Anpassung an den Defekt des Knochens dem Endbenutzer (z. B. Operateur) erleichtert wird, z. B. durch Schnitzen mit chirurgischen Instrumenten (z. B. Skalpell).

In einer weiteren Ausführungsform erfolgt die vollständige Aushärtung des strukturierten mineralischen Knochenersatzformkörpers mit verbleibender hydraulischer Aktivität - nach der Initiation und des Abbruchs des Abbindeprozesses - durch erneutes Kontaktieren mit einer wässrigen Flüssigkeit, enthaltend biologische Komponenten und/oder pharmakologische Wirkstoffe und/oder isolierte oder kultivierte Zellen.

Vorteilhaft erfolgt das erfindungsgemäße Verfahren zur Herstellung strukturierter mineralischer Knochenersatzformkörper unter moderaten Bedingungen (Temperaturen zwischen 0°C und 150°C, bevorzugt zwischen 25°C und 75°C), wodurch sich die strukturierten mineralischen Knochenersatzformkörper besonders als Träger für pharmakologische Wirkstoffe eignen, da temperatursensitive pharmakologische Wirkstoffe, wie Peptide und Proteine, Nukleinsäuren, oder Zellpräparationen, erhalten bleiben. Weiterhin vorteilhaft werden unvorhersehbare Reaktionen zwischen Calciumphosphaten und Strontiumverbindungen durch die moderaten Bedingungen verhindert.

Vorteilhaft wird durch das erfindungsgemäße Verfahren eine hohe Maßhaltigkeit der Formkörper erreicht, wobei weder Strangbrüche noch Oberflächenveränderungen an den Einzelsträngen auftreten.

Ein weiterer Aspekt der Erfindung betrifft die Verwendung der erfindungsgemäßen strukturierten mineralischen Knochenersatzformkörper zur Herstellung eines alloplastischen Implantats.

Unter alloplastisches Implantat wird ein Implantat aus körperfremden, künstlichen Materialien verstanden.

Ebenfalls hierin beschrieben, aber nicht in den Anwendungsbereich der beanspruchten Erfindung fallend, ist die Verwendung des erfindungsgemäßen strukturierten mineralischen Knochenersatzformkörpers als alloplastisches Implantatmaterial für die Auffüllung von Knochendefekten. Knochendefekte können angeboren sein oder als Folge von Unfällen auftreten, nach chirurgischen Eingriffen, insbesondere nach der Entfernung von Knochentumoren, Zysten, Prothesenwechseln, Umstellungsosteotomien, etc..

Ebenfalls hierin beschrieben, aber nicht in den Anwendungsbereich der beanspruchten Erfindung fallend, ist die Verwendung der erfindungsgemäßen strukturierten mineralischen Knochenersatzformkörper in der Mund-/Kieferchirurgie zum Aufbau neuen Knochens, z. B. Kieferkammaufbau, Sinuslift oder Füllung von Extraktionshöhlen. Ebenfalls hierin beschrieben, aber nicht in den Anwendungsbereich der beanspruchten Erfindung fallend, ist die Verwendung der erfindungsgemäßen strukturierten mineralischen Knochenersatzformkörper in der Orthopädie, Unfallchirurgie und Wirbelsäulenchirurgie zur Auffüllung von Knochendefekten aller Art.

Vorteilhaft weisen die erfindungsgemäßen strukturierten mineralischen Knochenersatzformkörper gegenüber anderen derzeit verwendeten biologischen und alloplastischen Knochenersatzmaterialien eine definierte Porosität, eine erhöhte Bioaktivität, definierte mechanische Eigenschaften, eine einfache intra-operative Bearbeitbarkeit und eine gute Kombinierbarkeit mit biologischen Substanzen, Knochenzellen und pharmakologischen Wirkstoffen, auf.

In einer weiteren Ausführungsform erfolgt die Verwendung der erfindungsgemäßen strukturierten mineralischen Knochenersatzformkörper als Trägermaterial in der Zellkultur, bevorzugt für die Kultivierung von Knochenzellen, der Gewebekultur und/oder des *Tissue Engineering ex vivo.*

Unter *Tissue Engineering* wird die Gewebekonstruktion oder auch Gewebezüchtung verstanden, bei der künstlich durch eine gerichtete Kultivierung von Zellen biologisches Gewebe hergestellt werden soll.

In einer weiteren Ausführungsform erfolgt die Verwendung der erfindungsgemäßen strukturierten mineralischen Knochenersatzformkörper als Trägermaterialien in der Biotechnologie, bevorzugt für die Kultivierung von Bakterien- oder Hefezellen, welche bereits im Herstellungsprozess in das Trägermaterial eingebracht werden können.

In einer weiteren Ausführungsform erfolgt die Verwendung der erfindungsgemäßen strukturierten mineralischen Knochenersatzformkörper als Trägermaterial für das *Tissue Engineering ex vivo,* wobei der erfindungsgemäße strukturierte mineralische Knochenersatzformkörper *in vitro* mit Knochenzellen, bevorzugt autologen Knochenzellen oder Stammzellen, unter sterilen Bedingungen besiedelt und inkubiert wird, wobei die Zellen vermehrt werden können. Nach Abschluss der Kultivierung wird ein autologisiertes Knochenersatzimplantat erhalten, das insbesondere bei der Regeneration großer Knochendefekte indiziert ist.

Unter autolog wird "zu demselben Individuum gehörig" verstanden.

In einer weiteren Ausführungsform erfolgt die Verwendung der erfindungsgemäßen strukturierten mineralischen Knochenersatzformkörper als Trägermaterial für das *Tissue Engineering ex vivo* in einem Bioreaktor.

Vorteilhaft weisen die erfindungsgemäßen strukturierten mineralischen Knochenersatzformkörper für diese Verwendung gute Voraussetzungen auf, insbesondere - neben den oben genannten Eigenschaften - die variable Formgebung ohne technologische Größenbeschränkung bei gleichzeitig vollständigem interkonnektierenden Porensystem, das eine entscheidende Voraussetzung für die gute Perfusion mit Kulturmedium im Bioreaktor ist.

Eine weitere Anwendung ist die Reinigung von (Ab-)Wasser von Schwermetallen und organischen Substanzen.

Für die Realisierung der Erfindung ist es auch zweckmäßig, die vorbeschriebenen Ausführungsformen und Merkmale der Ansprüche zu kombinieren.

### Ausführungsbeispiele

Nachfolgend soll die Erfindung anhand einiger Ausführungsbeispiele und zugehöriger Figuren eingehender erläutert werden. Die Ausführungsbeispiele sollen dabei die Erfindung beschreiben, ohne diese zu beschränken.

Es zeigen die
**Fig. 1** eine Abbildung eines zylindrischen und keilförmigen Knochenersatzformkörpers,
**Fig. 2** das Ergebnis der statistischen histomorphometrischen Auswertung der knöchernen Integration von strukturierten mineralischen Knochenersatzformkörpern (Referenzbeispiel) und strukturierten mineralischen Knochenersatzformkörpern mit Strontiumverbindungen nach 6 Monaten Implantationszeit.

### Herstellung der strukturierten mineralischen Knochenersatzformkörper

### Anmischen eines reaktiven mineralischen Knochenzementes ohne Strontiumverbindungen (Referenzbeispiel):

Die Herstellung des reaktiven mineralischen Knochenzementes erfolgte durch Mischen von 60 Gew.-% α-Tricalciumphosphat (α-Ca₃(PO₄)₂), 26 Gew.-% Calciumhydrogenphosphat (Monetit, CaHPO₄), 10 Gew.-% Calciumcarbonat (CaCO₃), 4 Gew.-% gefällten Hydroxylapatit (Ca₁₀(PO₄)₆(OH)₂) unter Zusatz von 2,5 Gew.-% fein gemahlenem Kaliumhydrogenphosphat (K₂HPO₄) und Herstellung einer Paste mit einem kurzkettigen/mittelkettigen Triglycerid (Miglyol 812, Cäsar und Lorenz, Hilden, Deutschland), Cremophor ELP (BASF, Ludwigshafen, Deutschland) und Hexadecylphosphat (Cetylphosphat, Amphisol A, Brenntag AG, Mühlheim an der Ruhr, Deutschland) mit einem Pulver-zu-Flüssigkeit-Verhältnis von 6:1.

### Anmischen eines reaktiven mineralischen Knochenzementes mit Strontiumverbindung:

Die Herstellung des reaktiven mineralischen Knochenzementes erfolgte durch Mischen von 60 Gew.-% α-Tricalciumphosphat (α-Ca₃(PO₄)₂), 26 Gew.-% Calciumhydrogenphosphat (Monetit, CaHPO₄), 10 Gew.-% Strontiumcarbonat (SrCO₃), 4 Gew.-% gefällten Hydroxylapatit (Ca₁₀(PO₄)₆(OH)₂) unter Zusatz von 2,5 Gew.-% fein gemahlenem Kaliumhydrogenphosphat (K₂HPO₄) und Herstellung einer Paste mit einem kurzkettigen/mittelkettigen Triglycerid Miglyol 812, Cremophor ELP und Hexadecylphosphat mit einem Pulver-zu-Flüssigkeit-Verhältnis von 6:1.

### Formgebung der Knochenzementmasse:

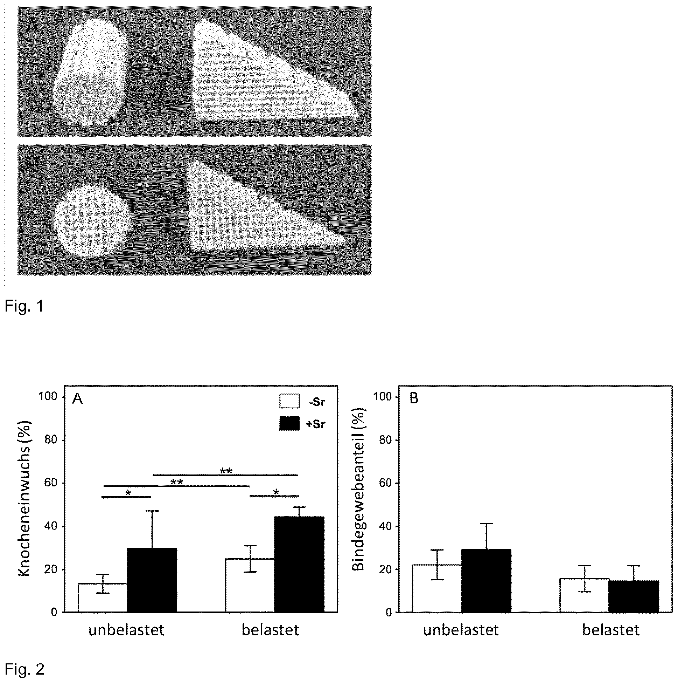

Die formbare Knochenzementmasse bzw. der reaktive mineralische Knochenzement wurde in 10 ml-Druckkartuschen mit einer 0,41 mm-Dosiernadel (Nordson, Dettingen unter Teck, Deutschland) gefüllt. Ein *Discovery* 3D-Drucker (regenHU, Villaz-St-Pierre, Schweiz) wurde zur Herstellung der strukturierten mineralischen Knochenersatzformkörper verwendet. Das Design des Knochenersatzformkörpers basiert auf der mäandrierenden Führung der Dosiernadel und damit Strangablage zur Herstellung des Grundrisses. Die dreidimensionale Ausdehnung erfolgte durch alternierende orthogonale Schichten, wobei zylindrische (Durchmesser von 9,2 mm, Höhe von 15 mm) und keilförmige (orthogonale Abmessungen von 24 mm und 14 mm, Höhe 6 mm) Knochenersatzformkörper hergestellt wurden (Fig. 1). Der Strangdurchmesser entspricht dem Durchmesser der Dosiernadel. Das Porensystem wurde durch Stangabstände von 0,59 mm festgelegt.

Fig. 1 zeigt in der seitlichen Ansicht (A) und Ansicht von oben (B) des Knochenersatzformkörpers die akkurate Strangablagerung und das definierte interkonnektierende Porensystem.

### Abbindeprozess:

Nach der Formgebung der Knochenzementmasse erfolgte die Inkubation in einer gesättigten Wasserdampfatmosphäre mit 95% relativer Luftfeuchtigkeit bei 50°C für 4 Tage und anschließender Inkubation in 0,9% Kochsalzlösung bei 37°C für 14 Tage zur Herstellung eines vollständig ausgehärteten strukturierten mineralischen Knochenersatzformkörpers.

### Abbruch des Abbindeprozesses:

Der Abbruch des Abbindeprozesses erfolgte durch dreimaliges Waschen der ausgehärteten Knochenersatzformkörper mit Aceton (99,5%) und anschließender Trocknung bei 50°C im Trockenschrank bei atmosphärischem Druck.

### Charakterisierung der strukturierten mineralischen Knochenersatzformkörper

### Bestimmung des Anteils an abgebundenem mineralischem Knochenzement mittels Röntgendiffraktometrie (XRD):

Der Grad der Umsetzung kann anhand des röntgendiffraktometrisch bestimmten Gehalts an α-TCP bestimmt werden. Nach vollständiger Aushärtung ist kein Peak für α-TCP im Röntgendiffraktogramm zu erkennen. Die Peaks für Calcit (CaCO₃) und Monetit (CaHPO₄) sind ebenfalls nicht mehr nachweisbar oder nur angedeutet zu erkennen.

### Bestimmung der Makroporosität:

Die Berechnung der Makroporosität erfolgt anhand der Dichte (CPC-Paste ca. 2,0 g/cm³) in Relation zu Masse und Volumen der Formkörper.

Die Makroporosität der strukturierten mineralischen Knochenersatzformkörper beträgt 50% bei einer Dichte der Formkörper von 1,0 g/cm³.

### Bestimmung der spezifischen Oberfläche mittels BET-Messung:

Die Bestimmung der spezifischen Oberfläche von strukturierten mineralischen Knochenersatzformkörpern erfolgte mittels Gasabsorptionsmethode nach Brunauer-Emmet-Teller (BET-Verfahren). Es wurden poröse Formkörper der Abmessung 20 mm x 10 mm x 10 mm mit einer Strangdicke von 0,33 mm analog zum oben beschriebenen Verfahren zur Formgebung und nachfolgenden Behandlung hergestellt. Die Formkörper wurden anschließend zerkleinert und als Probe in die BET-Messanlage überführt. Die Messung erfolgt vollautomatisch. Die Ergebnisse werden als Protokoll ausgegeben:

| | |
|---|---|
| Spezifische Oberfläche nach BET Probe 1: | 11,2238 m²/g |
| Spezifische Oberfläche nach BET Probe 2: | 12,4238 m²/g |
| Mittelwert: | 11,8238 m²/g |

### Bestimmung der Druckfestigkeit:

Zur Bestimmung der Druckfestigkeit der erfindungsgemäßen Knochenersatzformkörper wurde eine Universalprüfmaschine wie die der Fa. Hegewald & Peschke unter Verwendung einer Kraftmesszelle von 20 kN mit einer Vorschubgeschwindigkeit von 1 mm/min verwendet.

Die Druckfestigkeit der strukturierten mineralischen Knochenersatzformkörper ohne Strontiumverbindungen beträgt 10 MPa (Referenzbeispiel) und der erfindungsgemäßen strukturierten mineralischen Knochenersatzformkörper mit Strontiumverbindungen 18 MPa.

### In vivo-Versuche:

Die Tierstudie wurde vom lokalen Ethikausschuss (Regierungspräsidium Tübingen, Deutschland, No. 1142) genehmigt und ist in Übereinstimmung mit den internationalen Empfehlungen für Pflege und Verwendung von Labortieren (ARRIVE guidelines and EU Directive 2010/63/EU for animal experiments). Insgesamt wurden 14 weibliche Merinoschafe (3 bis 5 Jahre, 70 bis 110 kg) für die Studie verwendet. Jedes Schaf erhielt sowohl einen strukturierten mineralischen Knochenersatzformkörper ohne Strontiumverbindungen (Referenzbeispiel) als auch einen erfindungsgemäßen strukturierten mineralischen Knochenersatzformkörper mit Strontiumverbindungen, jeweils in eine hintere Gliedmaße. Die Knochenersatzformkörper wurden in einen Bohrdefekt im distalen Oberschenkelgelenkkopf (unbelastete Bedingungen) und in der Schienbeinmetaphyse (belastete Bedingungen) implantiert. Leere Defekte wurden weggelassen, da bereits gezeigt wurde, dass beide Defektmodelle eine kritische Größe aufweisen und nicht während der Untersuchung heilen (Kanter *et al.* 2016; Harms *et al.* 2012); und somit die Anzahl der Versuchstiere zu reduzieren. Nach 6 und 26 Wochen wurden die Schafe eingeschläfert (n = 7 pro Gruppe) und der Oberschenkelknochen und das Schienbein entnommen und für histologische und energiedispersive Röntgenspektroskopie (energy dispersive X-ray spectroscopy, EDX)-Analyse vorbereitet.

### Chirurgisches Vorgehen:

Sämtliche chirurgischen Eingriffe erfolgten unter Vollnarkose. Die Schafe wurden intramuskulär mit 0,2 mg/kg Xylazinhydrochlorid (Rompun®, 2%, Bayer Leverkusen, Deutschland) vorbehandelt. Die Narkose wurde mit einer intravenösen Injektion von 5,0 mg/kg Thiopental (Thiopental Inresa, Inresa GmbH, Freiburg, Deutschland) induziert und durch Isofluraninhalation (Forene®, Abbott GmbH, Wiesbaden, Deutschland) aufrechterhalten. Die Schafe erhielten 4,0 mg/kg Carprofen (Rimadyl®, Pfizer, Karlsruhe, Deutschland) zur Analgesie und 7,0 mg/kg Amoxicillintrihydrat (Veyxyl®, VeyxPharma GmbH, Schwarzenborn, Deutschland) subkutan zur antibiotischen Prophylaxe für 4 Tage.

Die strukturierten mineralischen Knochenersatzformkörper ohne Strontiumverbindungen (Referenzbeispiel) und die erfindungsgemäßen strukturierten mineralischen Knochenersatzformkörper mit Strontiumverbindungen wurden in die rechte bzw. linke hintere Gliedmaße implantiert. Das chirurgische Vorgehen erfolgte entsprechend Kanter *et al.* bzw. Harms *et al.* (Kanter *et al.* 2016; Harms *et al.* 2012), wobei ein Defekt mit einem Durchmesser von 10 mm und einer Tiefe von 15 mm in den trabekularen Knochen des Oberschenkelknochens gebohrt und ein keilförmiger Defekt (Breite von 14 mm, Länge von 24 mm, Höhe von 6 mm) in den trabekularen Knochen 3 mm unter der mittleren Schienbeinebene erzeugt wurde. Die Defekte wurden sorgfältig von Knochenteilen durch Spülen mit steriler Kochsalzlösung gereinigt und die Knochenersatzformkörper eingesetzt.

Zur Markierung neu gebildeten Knochens wurden Tetracyclinhydrochlorid (25 mg/kg Körpergewicht) und Calceingrün (10 mg/kg Körpergewicht) 2 bzw. 4 Wochen nach der Implantation injiziert, wenn die Schafe nach 6 Wochen eingeschläfert wurden, und 8 bzw. 16 Wochen nach der Implantation injiziert, wenn die Schafe nach 26 Wochen eingeschläfert wurden.

### Histologische Untersuchungen:

Die Implantate und etwa 10 mm des umgebenden Knochens wurden entnommen und für die nicht-entkalkte Histologie vorbereitet. Die Proben wurden in 4%-iger gepufferter Formaldehydlösung fixiert, in einer Serie von Lösungen mit steigendem Ethanolgehalt dehydriert und eingebettet in Methacrylat (Merck KGaA, Darmstadt, Deutschland). 70 µm-Schnitte wurden angefertigt und mit Paragon (Paragon C&C, New York, NY, US) entsprechend Standardprotokollen gefärbt. Die Proben wurden mit 50-facher Vergrößerung unter dem Lichtmikroskop (Leica DM16000B, Software MMAF Version 1.4.0 MetaMorph®, Leica, Heerbrugg, Schweiz) untersucht und im Blindversuch ausgewertet. Zur Bildanalyse wurden Photoshop (Adobe Systems, Mountain View, CA, US) und MATLAB® (MATLAB R2009b, TheMathsWorks Inc., Natick, MA, US) verwendet. Die Fläche des Knochenersatzformkörpers, des Knochens und des Bindegewebes sowie die relative Implantatoberfläche, welche in direktem Knochenkontakt steht, wurden bestimmt.

Osteoklasten wurden durch histologische Färbung der Tartrat-resistenten sauren Phosphatase *(tartrate-resistant acid phosphatase,* TRAP), nach der Entkalkung der Proben und Einbettung in Paraffin entsprechend Recknagel *et al.* (Recknagel *et al.* 2013), identifiziert. Zellen mit mindestens 3 Zellkernen, welche sich auf dem Knochen oder den Knochenersatzformkörper befinden und positiv für die TRAP-Färbung waren, wurden als Osteoklasten definiert. Die Zellen wurden in 6 sichtbaren Gebieten (etwa 8 mm²) unter 100-facher Vergrößerung zentral auf dem Implantat gezählt. Die räumliche Verteilung des Tetracyclin- und Calcein-markierten Knochens wurde analysiert unter Fluoreszenzlicht zur qualitativen Bestimmung des zeitlichen Knocheneinwachsens.

### Energiedispersive Röntgenspektroskopie (EDX):

Die Bestimmung des Strontiumgehalts erfolgte durch Schleifen der Methacrylat-eingebetteten Proben und Beschichtung mit Kohlenstoff (MED 010, Balzers Union, Dietenheim, Deutschland). Die EDX-Analyse wurde mit einem Zeiss DSM 962 Rasterelektronenmikroskop (Zeiss, Jena, Deutschland) und Genesis Line/Map 6.44-Software (EDAX, Weiterstadt, Deutschland) durchgeführt. Die Spektralkarten wurden mit einer Beschleunigungsspannung von 20kV aufgenommen.

### Statistik:

Unterschiede zwischen der 6 Wochen- und 26 Wochen-Gruppe wurden durch die Verwendung des parameterfreien Mann-Whitney-U-Tests identifiziert. Zur Bestimmung der Unterschiede innerhalb einer Implantationsperiode wurde die Signifikanz durch den Wilcoxon-Vorzeichen-Rang-Test bestimmt. Die Statistik wurde mittels der SPSS-Software (v21, SPSS Inc., Chicago, Illinois, US) durchgeführt. Das Signifikanzniveau wurde auf p ≤ 0,05 festgelegt.

Fig. 2 zeigt das Ergebnis der statistischen histomorphometrischen Auswertung der knöchernen Integration von strukturierten mineralischen Knochenersatzformkörpern (Referenzbeispiel) und strukturierten mineralischen Knochenersatzformkörpern mit Strontiumverbindungen nach 6 Monaten Implantationszeit: Neuer Knocheneinwuchs (A) und Bindegewebeanteil (B) in strukturierten mineralischen Knochenersatzformkörpern (Referenzbeispiel) (weiße Säulen) und strukturierten mineralischen Knochenersatzformkörpern mit Strontiumverbindungen (schwarze Säulen) unter belasteten und unbelasteten Bedingungen. (*, **) jeweils signifikanter Unterschied p < 0.05.

### Zitierte Nichtpatentliteratur

M. P. Hofmann, U. Gbureck, C. O. Duncan, M. S. Dover, J. E. Barralet (2007) Carvable calcium phosphate bone substitute material. J. Biomed. Mater. Res. B Appl. Biomater., 83B, 1, 1-8.
A. Lode, K. Meissner, Y. Luo, F. Sonntag, S. Glorius, B. Nies, C. Vater, F. Despang, T. Hanke, M. Gelinsky (2014) Fabrication of porous scaffolds by three-dimensional plotting of a pasty calcium phosphate bone cement under mild conditions. J. Tissue Eng. Regen. Med., 8, 682-693. E. W. Washburn (1921) The Dynamics of Capillary Flow. Physical Review Band, 17, Nr. 3, 273-283.
B. Kanter, M. Geffers, A. Ignatius, U. Gbureck (2016) Control of in vivo mineral bone cement degradation. Acta Biomater., 10, 3279-3287.
C. Harms, K. Helms, T. Taschner, I. Stratos, A. Ignatius, T. Gerber et al. (2012) Osteogenic capacity of nanocrystalline bone cement in a weight-bearing defect at the ovine tibial metaphysis. Int. J. Nanomedicine. 7, 2883-2889.
S. Recknagel, R. Bindl, C. Brochhausen, M. Gockelmann, T. Wehner, P. Schoengrafetal. (2013) Systemic inflammation induced by a thoracic trauma alters the cellular composition of the early fracture callus. J. Trauma. Acute Care Surg., 74, 531-537.

## Patentansprüche

1. Strukturierte mineralische Knochenersatzformkörper mit einem definierten interkonnektierenden Porensystem und einer vorbestimmten Struktur,
hergestellt aus einem wasserfreien mineralischen Knochenzement enthaltend Calcium- und/oder Magnesiumverbindungen und 0,5 Mol-% bis 25 Mol-% Strontiumionen bezogen auf den Gesamtgehalt zweiwertiger Kationen,
wobei das definierte interkonnektierende Porensystem in mindestens einer Dimension ein interkonnektierendes Porensystem mit Poren mit einem durchschnittlichen Porenquerschnitt von mindestens 50.000 µm² und einem durchschnittlichen Porendurchmesser von mindestens 250 µm bezogen auf einen kreisrunden Querschnitt aufweist, wobei das definierte interkonnektierende Porensystem in mindestens einer Dimension ein interkonnektierendes Porensystem mit Poren mit einem maximalen durchschnittlichen Porenquerschnitt von 785.000 µm² und einem maximalen durchschnittlichen Porendurchmesser von 1000 µm bezogen auf einen kreisrunden Querschnitt aufweist.

2. Strukturierte mineralische Knochenersatzformkörper nach Anspruch 1, **dadurch gekennzeichnet, dass** die strukturierten mineralischen Knochenersatzformkörper eine Gesamtporosität zwischen 50% und 85% aufweisen.

3. Strukturierte mineralische Knochenersatzformkörper nach Anspruch 1 oder 2, **dadurch gekennzeichnet, dass** die strukturierten mineralischen Knochenersatzformkörper eine spezifische Oberfläche von mindestens 5 m²/g aufweisen.

4. Strukturierte mineralische Knochenersatzformkörper nach einem der Ansprüche 1 bis 3, **dadurch gekennzeichnet, dass** die strukturierten mineralischen Knochenersatzformkörper 2 Mol-% bis 20 Mol-% Strontiumionen bezogen auf den Gesamtgehalt zweiwertiger Kationen aufweisen.

5. Strukturierte mineralische Knochenersatzformkörper nach einem der Ansprüche 1 bis 4, **dadurch gekennzeichnet, dass** das definierte interkonnektierende Porensystem in mindestens zwei Dimensionen ein interkonnektierendes Porensystem mit Poren mit einem maximalen durchschnittlichen Porenquerschnitt von 785.000 µm² und einem maximalen durchschnittlichen Porendurchmesser von 1000 µm bezogen auf einen kreisrunden Querschnitt aufweist.

6. Verfahren zur Herstellung strukturierter mineralischer Knochenersatzformkörper, umfassend die folgenden Schritte:
a. Anmischen eines reaktiven mineralischen Knochenzementes umfassend Calcium- und/oder Magnesiumverbindungen und 0,5 Mol-% bis 25 Mol-% Strontiumverbindungen bezogen auf den Gesamtgehalt zweiwertiger Kationen mit einer wasserfreien Trägerflüssigkeit zu einer formbaren Knochenzementmasse,
b. Formgebung der Knochenzementmasse durch ein 3D-Druckverfahren zu einem strukturierten mineralischen Knochenersatzformkörper mit einer vorbestimmten Struktur und einem definierten interkonnektierenden Porensystem,
wobei der Durchmesser der Stränge der Knochenzementmasse zwischen 200 µm und 1200 µm beträgt und
wobei der Abstand der Stränge der Knochenzementmasse zwischen 250 und 1000 µm beträgt,
c. Initiation des Abbindeprozesses durch Kontaktieren des Knochenersatzformkörpers mit einer wässrigen Lösung oder einer gesättigten Wasserdampfatmosphäre,
d. Abbruch des Abbindeprozesses durch den substantiellen Entzug von Wasser.

7. Verfahren nach Anspruch 6, **dadurch gekennzeichnet, dass** als reaktiver mineralischer Knochenzement mindestens eine Substanz aus der Gruppe der Silikate, Phosphate, Sulfate, Carbonate, Oxide und/oder Hydroxide in Verbindung mit Calciumionen und/oder Magnesiumionen und Strontiumionen verwendet wird.

8. Verfahren nach Anspruch 6 oder 7, **dadurch gekennzeichnet, dass** als reaktiver mineralischer Knochenzement mindestens ein Phosphat in Verbindung mit Calciumionen verwendet wird, wobei das molare Verhältnis Calcium zu Phosphat mindestens 1 und das molare Verhältnis zweiwertiger Kationen zu Phosphationen mindestens 1,35 ist.

9. Verfahren nach einem der Ansprüche 6 bis 8, **dadurch gekennzeichnet, dass** die wässrige Lösung zur Initiation des Abbindeprozesses mindestens einen Zusatzstoff ausgewähltaus einer Pufferlösung, einem organischen und/oder anorganischen Salz, einem Protein, einer Zellpräparation, einem biologischen, rekombinanten oder pharmakologischen Wirkstoff, einer Nukleinsäure, einer Mischung von Nukleinsäuren, einer Aminosäure, einer modifizierten Aminosäure, einem Vitamin und einer Mischung aus diesen enthält.

10. Verfahren nach einem der Ansprüche 6 bis 9, **dadurch gekennzeichnet, dass** die Initiation des Abbindeprozesses in einer gesättigten Wasserdampfatmosphäre bei mindestens 90% relativer Luftfeuchtigkeit und einer Temperatur zwischen 0°C und 150°C erfolgt.

11. Verfahren nach einem der Ansprüche 6 bis 10, **dadurch gekennzeichnet, dass** der Abbruch des Abbindeprozesses durch den substantiellen Entzug von Wasser durch ein Trocknungsverfahren oder durch einen Austausch des Wassers gegen mindestens ein leicht flüchtiges, toxikologisch unbedenkliches Lösungsmittel erfolgt.

12. Verwendung eines strukturierten mineralischen Knochenersatzformkörpers nach einem der Ansprüche 1 bis 5 zur Herstellung eines alloplastischen Implantats.

13. Verwendung eines strukturierten mineralischen Knochenersatzformkörpers nach einem der Ansprüche 1 bis 5 als Trägermaterial in der Zellkultur und/oder der Gewebekultur.

14. Verwendung eines strukturierten mineralischen Knochenersatzformkörpers nach einem der Ansprüche 1 bis 5 als Trägermaterial des Tissue Engineering *ex vivo.*

## Claims

1. Structured mineral bone substitute moldings having a defined interconnecting pore system and a predetermined structure,
made of a anhydrous mineral bone cement containing calcium and/or magnesium compounds and from 0.5 Mol-% to 25 Mol-% strontium ions, based on the total content of divalent cations,
wherein the defined interconnecting pore system in at least one dimension has an interconnecting pore system having pores that have an average pore cross section of at least 50,000 µm² and an average pore diameter of at least 250 µm, based on a circular cross section, wherein the defined interconnecting pore system in at least one dimension has an interconnecting pore system having pores that have a maximum average pore cross section of 785,000 µm² and a maximum average pore diameter of 1000 µm, based on a circular cross-section.

2. Structured mineral bone substitute moldings of claim 1, **characterized in that** the structured mineral bone substitute moldings have a total porosity of between 50 % and 85 %.

3. Structured mineral bone substitute moldings of claim 1 or 2, **characterized in that** the structured mineral bone substitute moldings have a specific surface area of at least 5 m²/g.

4. Structured mineral bone substitute moldings of one of the claims 1 to 3, **characterized in that** the structured mineral bone substitute moldings comprise from 2 Mol-% to 20 Mol-% strontium ions, based on the total content of divalent cations.

5. Structured mineral bone substitute moldings of one of the claims 1 to 4, **characterized in that** the defined interconnecting pore system in at least two dimensions has an interconnecting pore system having pores that have a maximum average pore cross section of 785,000 µm² and a maximum average pore diameter of 1000 µm, based on a circular cross section.

6. Method for producing structured mineral bone substitute moldings, comprising the following steps:
a. mixing a reactive mineral bone cement, comprising calcium and/or magnesium compounds and from 0.5 Mol-% to 25 Mol-% strontium compounds, based on the total content of divalent cations, with an anhydrous carrier liquid to form a moldable bone cement mixture;
b. shaping the bone cement mixture into a structured mineral bone substitute molding, which has a predetermined structure and a defined interconnecting pore system, by means of 3D printing,
wherein the diameter of the strands of the bone cement mixture is between 200 µm and 1200 µm, and
wherein the distance between the strands of the bone cement mixture is between 250 µm and 1000 µm;
c. initiating the setting process by bringing the bone substitute molding into contact with an aqueous solution or a saturated water vapor atmosphere; and
d. terminating the setting process by the substantial removal of water.

7. Method of claim 6, **characterized in that** as reactive mineral bone cement at least one substance from the group consisting of silicates, phosphates, sulfates, carbonates, oxides and/or in combination with calcium ions and/or magnesium ions and strontium ions hydroxides is used.

8. Method of claim 6 or 7, **characterized in that** as reactive mineral bone cement at least one phosphate in combination with calcium ions is used, wherein the molar ratio of calcium to phosphate being at least 1, and the molar ratio of divalent cations to phosphate ions being at least 1.35.

9. Method of one of the claims 6 to 8, **characterized in that** the aqueous solution for initiating the setting process comprises at least one additive selected from a buffer solution, an organic and/or inorganic salt, a protein, a cell preparation, a biological, recombinant or pharmacological active ingredient, a nucleic acid, a mixture of nucleic acids, an amino acid, a modified amino acid, a vitamin and a mixture thereof.

10. Method of one of the claims 6 to 9, **characterized in that** the setting process is initiated in a saturated water vapor atmosphere at a relative humidity of at least 90 % and a temperature of between 0 °C and 150 °C.

11. Method of one of the claims 6 to 10, **characterized in that** the setting process is terminated by the substantial removal of water using a drying process or by replacing the water with at least one volatile, toxicologically safe solvent.

12. Use of a structured mineral bone substitute molding according to one of the claims 1 to 5 for producing an alloplastic implant.

13. Use of a structured mineral bone substitute molding according to one of the claims 1 to 5 as a carrier material in cell culturing and/or tissue culturing.

14. Use of a structured mineral bone substitute molding according to one of the claims 1 to 5 as a carrier material in tissue engineering *ex vivo.*

## Revendications

1. Éléments moulés structurés minéraux de substitution osseuse comportant un système de pores interconnecté défini et une structure prédéterminée, produits à partir d'un ciment osseux minéral anhydre contenant des composés de calcium et/ou de magnésium et 0,5 % en mol à 25 % en mol d'ions strontium par rapport à la teneur totale en cations divalents, le système de pores interconnecté défini présentant, dans au moins une dimension, un système de pores interconnecté comportant des pores comportant une section transversale de pore moyenne d'au moins 50 000 µm² et un diamètre de pore moyen d'au moins 250 µm par rapport à une section transversale circulaire, le système de pores interconnecté défini présentant, dans au moins une dimension, un système de pores interconnecté comportant des pores comportant une section transversale de pore moyenne maximale de 785 000 µm² et un diamètre de pore moyen maximal de 1 000 µm par rapport à une section transversale circulaire.

2. Éléments moulés structurés minéraux de substitution osseuse selon la revendication 1, **caractérisés en ce que** les éléments moulés structurés minéraux de substitution osseuse présentent une porosité totale comprise entre 50 % et 85 %.

3. Éléments moulés structurés minéraux de substitution osseuse selon la revendication 1 ou 2, **caractérisés en ce que** les éléments moulés structurés minéraux de substitution osseuse présentent une superficie spécifique d'au moins 5 m²/g.

4. Éléments moulés structurés minéraux de substitution osseuse selon l'une des revendications 1 à 3, **caractérisés en ce que** les éléments moulés structurés minéraux de substitution osseuse présentent 2 % en mol à 20 % en mol d'ions strontium par rapport à la teneur totale en cations divalents.

5. Éléments moulés structurés minéraux de substitution osseuse selon l'une des revendications 1 à 4, **caractérisés en ce que** le système de pores interconnecté défini présente, dans au moins deux dimensions, un système de pores interconnecté comportant des pores comportant une section transversale de pore moyenne maximale de 785 000 µm² et un diamètre de pore moyen maximal de 1 000 µm par rapport à une section transversale circulaire.

6. Procédé de production d'éléments moulés structurés minéraux de substitution osseuse, comprenant les étapes suivantes :
a. mélange d'un ciment osseux minéral réactif comprenant des composés de calcium et/ou de magnésium et 0,5 % en mol à 25 % en mol de composés de strontium par rapport à la teneur totale en cations divalents avec un liquide porteur anhydre pour former une masse de ciment osseux malléable,
b. moulage de la masse de ciment osseux par un procédé d'impression 3D en un élément moulé structuré minéral de substitution osseuse comportant une structure prédéterminée et un système de pores interconnecté défini,
le diamètre des brins de la masse de ciment osseux étant compris entre 200 µm et 1 200 µm,
et la distance entre les brins de la masse de ciment osseux étant comprise entre 250 et 1 000 µm,
c. initiation du processus de prise par la mise en contact de l'élément moulé de substitution osseuse avec une solution aqueuse ou une atmosphère de vapeur d'eau saturée,
d. arrêt du processus de prise par l'élimination substantielle d'eau.

7. Procédé selon la revendication 6, **caractérisé en ce qu'**au moins une substance du groupe des silicates, phosphates, sulfates, carbonates, oxydes et/ou hydroxydes est utilisée en combinaison avec des ions calcium et/ou des ions magnésium et des ions strontium en tant que ciment osseux minéral réactif.

8. Procédé selon la revendication 6 ou 7, **caractérisé en ce qu'**au moins un phosphate est utilisé en combinaison avec des ions calcium en tant que ciment osseux minéral réactif, le rapport molaire entre le calcium et le phosphate étant d'au moins 1 et le rapport molaire entre les cations divalents et les ions phosphate étant d'au moins 1,35.

9. Procédé selon l'une des revendications 6 à 8, **caractérisé en ce que** la solution aqueuse contient, pour l'initier le processus de prise, au moins un additif choisi parmi une solution tampon, un sel organique et/ou inorganique, une protéine, une préparation cellulaire, un principe actif biologique, recombinant ou pharmacologique, un acide nucléique, un mélange d'acides nucléiques, un acide aminé, un acide aminé modifié, une vitamine et un mélange de ceux-ci.

10. Procédé selon l'une des revendications 6 à 9, **caractérisé en ce que** l'initiation du processus de prise est effectuée dans une atmosphère de vapeur d'eau saturée à une humidité relative d'au moins 90 % et à une température comprise entre 0 °C et 150 °C.

11. Procédé selon l'une des revendications 6 à 10, **caractérisé en ce que** l'arrêt du processus de prise est effectué par l'élimination substantielle d'eau par un procédé de séchage ou par un échange de l'eau contre au moins un solvant volatil, inoffensif sur le plan toxicologique.

12. Utilisation d'un élément moulé structuré minéral de substitution osseuse selon l'une des revendications 1 à 5 pour la production d'un implant alloplastique.

13. Utilisation d'un élément moulé structuré minéral de substitution osseuse selon l'une des revendications 1 à 5 en tant que matériau porteur dans la culture cellulaire et/ou la culture tissulaire.

14. Utilisation d'un élément moulé structuré minéral de substitution osseuse selon l'une des revendications 1 à 5 en tant que matériau porteur de l'ingénierie tissulaire *ex vivo.*
